# EUROPÄISCHE PATENTSCHRIFT

(11) **EP 2 089 387 B1**
(45) Veröffentlichungstag und Bekanntmachung des Hinweises auf die Patenterteilung: **23.10.2013**
(21) Anmeldenummer: 07819851.2
(22) Anmeldetag: 17.11.2007
(51) Int. Cl.: C07D 471/04, A61K 31/437, A61P 3/10

(54) **INDOLIZINDERIVATE UND IHRE VERWENDUNG ALS ANTIDIABETIKA**
INDOLIZINE DERIVATIVES AND THEIR USE AS ANTIDIABETICS
DÉRIVÉS D'INDOLIZINE ET LEUR UTILISATION COMME AGENTS ANTIDIABÉTIQUES

(30) Priorität: 11.12.2006 DE 102006058236
(43) Veröffentlichungstag der Anmeldung: 19.08.2009
(73) Patentinhaber: Merck Patent GmbH, 64293 Darmstadt (DE)
(72) Erfinder: MEDERSKI, Werner, 64673 Zwingenberg (DE); BEIER, Norbert, 64354 Reinheim (DE); BURGDORF, Lars Thore, 60389 Frankfurt am Main (DE); GERICKE, Rolf, 64342 Seeheim-Jugenheim (DE); KLEIN, Markus, 64291 Darmstadt (DE); TSAKLAKIDIS, Christos, 69469 Weinheim (DE)
(86) Internationale Anmeldenummer: PCT/EP2007/009961
(87) Internationale Veröffentlichungsnummer: WO 2008/071288

(56) Entgegenhaltungen:
- WO-A-2006/094235

## Beschreibung

Die Erfindung betrifft Verbindungen der Formel I 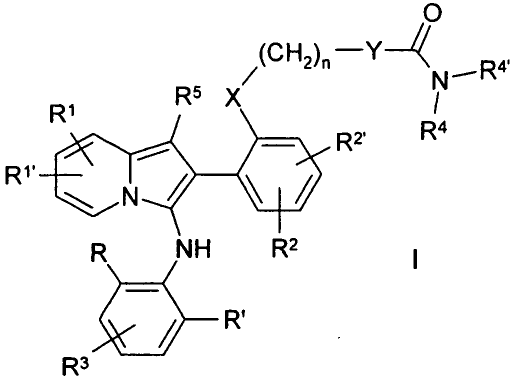 worin
- R, R': jeweils unabhängig voneinander A, OA oder Hal,
- R¹, R^{1'}: jeweils unabhängig voneinander H, A, F oder Cl,
- R², R^{2'}: jeweils unabhängig voneinander H, Hal, A, OH, OA, CN, NO₂, NR⁴R^{4'}, CH₂NR⁴R^{4'}, O(CH₂)ₘNR⁴R^{4'}, O(CH₂)ₘOR⁴, NH(CH₂)ₘNR⁴R^{4'}, O(C=O)(CH₂)ₘNR⁴R^{4'}, NH(C=O)(CH₂)ₘNR⁴R^{4'}, CH₂O(CH₂)ₘNR⁴R^{4'}, CH₂OR⁴ oder COOR⁴,
- R³: H, A oder Hal,
- R⁴, R^{4'}: jeweils unabhängig voneinander H oder A,
- R⁵: CN, COOA oder COOH,
- X, Y: jeweils unabhängig voneinander O, NH, CH₂ oder fehlt,
- A: unverzweigtes oder verzweigtes Alkyl mit 1-10 C-Atomen, worin 1-7 H-Atome durch F ersetzt sein können, oder Cycloalkyl mit 3-7 C-Atomen,
- Hal: F, Cl, Br oder I,
- m: 1, 2 oder 3,
- n: 0, 1 oder 2,
bedeuten,
sowie ihre pharmazeutisch verwendbaren Solvate, Salze und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Sie zeigen SGLT1 und SGLT2 (sodium dependent glucose co-transporter) inhibierende Eigenschaften und können daher zur Bekämpfung und Verhütung von Diabetes vom Typ 1 und Typ 2 eingesetzt werden.

Die Absorption von Glucose im Bürstensaum des Dünndarms und in den proximalen Nierentubuli gegen einen Konzentrationsgradienten erfolgt über epitheliale natriumabhängige Glucose-Cotransporter (SGLTs). Es wurden mindestens zwei größere Klassen von SGLTs beschrieben: SGLT1 (beispielsweise Lee W.S. et al. (1994) The high-affinity Na+/Glucose co-transporter: reevaluation of function and distribution of expression. J. Biol. Chem. 269, 12032-12039) und SGLT2 (beispielsweise Mackenzie B. et al. (1994) SAAT1 ist a low-affinity Na+/glucose cotransporter and not an amino acid transporter. J. Biol. Chem. 269, 22488-22491).
Es wird angenommen, dass SGLT1 für die Absorption von Glucose im Darm wichtig ist, wohingegen SGLT2 wahrscheinlich für die Reabsorption von frei filtrierter Glucose in der Niere hauptsächlich verantwortlich ist.

Die hauptsächliche Veränderung bei Diabetes mellitus ist Hyperglykämie. Dies ist nicht nur ein Symptom der Erkrankung, sondern auch ein potentieller pathogener Faktor, der zu multiplen chronischen diabetischen mikro- und makrovaskularen Komplikationen und einer Störung der Insulinsekretion und Empfindlichkeit führt (Klein R. (1995), Hyperglycemia and microvascular and macrovascular disease in diabetes, Diabetes Care 18, 258-268; Rossetti L. (1995), Glucose toxicity: the implications of hyperglycemia in the pathophysiology of diabetes mellitus, Clin. Invest. Med. 18, 255-260). Somit ist beim Diabetes-Patient die ausschließliche Regulation der Blut-Glucosespiegel innerhalb des normalen Bereichs ein wichtiges Therapieziel. Entsprechend ihrer beschriebenen Funktion führt eine Hemmung der SGLTs zu einer verringerten Absorption und gesteigerten Ausscheidung von Glucose, sowie zu einer anschließenden Abnahme der Blut-Glucosespiegel. Somit kann die Unterdrückung der SGLTs eine geeignete Alternative zur Behandlung von Diabetes sein.

In der Literatur sind mehrere Substanzklassen mit SGLT-Wirkung beschrieben. All diesen Strukturen diente als Leitbild der Naturstoff Phlorizin.
Aromatische Glycosidderivate kennt man aus WO 2004/052902 und WO 2004/052903. Propiophenonglycoside sind beschrieben in WO 0280936, WO 0280935, JP 2000080041 und EP 850948. Glucopyranoslyoxybenzylbenzole sind in WO 0244192, WO 0228872 und WO 0168660 beschrieben. Glucopyranosyloxy-pyrazole kennt man aus WO 0268440, WO 0268439, WO 0236602 und WO 0116147. O-Glycosidbenzamide sind in WO 0174835 und WO 0174834 offenbart. C-Arylglycoside sind in WO 0127128 und US 2002137903 beschrieben. Alle bekannten Strukturen enthalten als sehr wichtiges Strukturelement die Glucose. Ferner sind aus US 2002/132807 Diarylsulfid-Verbindungen zur Behandlung von Entzündungs- und Immun-Erkrankungen bekannt. In EP 0 953 357 A1 werden allgemein Glycosid-Verbindungen als renale Drug-Carrier und in WO 95/23780 4-Hydroxy-phenoxy-hetero-cycloalkyl-Verbindungen als Hautaufheller beschrieben.
Andere Indolizinderivate kennt man aus WO 2004/108722 und aus Bioorg. Med. Chem. Lett. 2006, 16, 3998ff, als auch aus WO 2006/094235.

Die erfindungsgemäßen Verbindungen weisen ein hohes Splitting in bezug auf die gewünschte Affinität von SGLT₂ zu SGLT₁ auf.

Die Verbindungen der Formel I zeichnen sich durch günstige Wirkungen auf den Glucosestoffwechsel aus, sie senken insbesondere den Blutzuckerspiegel und sind zur Behandlung von Typ 1 und Typ 2 Diabetes geeignet. Die Verbindungen können daher allein oder in Kombination mit weiteren Blutzucker-senkenden Wirkstoffen (Antidiabetika) eingesetzt werden.

Die Verbindungen der Formel I eignen sich weiterhin zur Prävention und Behandlung von diabetischen Spätschäden, wie z.B. Nephropathie, Retinopathie, Neuropathie sowie Syndrom X, Obesitas, Herzinfarkt, myocardialem Infarkt, peripheren arteriellen Verschlusskrankheiten, Thrombosen, Arteriosklerose, Entzündungen, Immunkrankheiten, Autoimmunkrankheiten, wie z.B. AIDS, Asthma, Osteoporose, Krebs, Psoriasis, Alzheimer, Schizophrenie und Infektionskrankheiten, bevorzugt ist die Behandlung von Typ 1 und Typ 2 Diabetes sowie zur Prävention und Behandlung 15 von diabetischen Spätschäden, Syndrom X und Obesitas.

Die Verbindungen der Formel I können als Arzneimittelwirkstoffe in der Human- und Veterinärmedizin eingesetzt werden, insbesondere zur Behandlung und Verhütung von Diabetes vom Typ 1 und Typ 2.

Gegenstand der Erfindung sind die Verbindungen der Formel I und ihre Salze sowie ein Verfahren zur Herstellung von Verbindungen der Formel I sowie ihrer pharmazeutisch verwendbaren Solvate, Salze und Stereoisomere, dadurch gekennzeichnet, daß man
a) eine Verbindung der Formel II 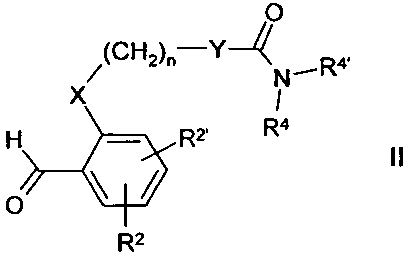 worin
   X, Y, R², R^{2'}, R⁴, R^{4'} and n die in Anspruch 1 angegebenen Bedeutungen haben,
   mit einer Verbindung der Formel III 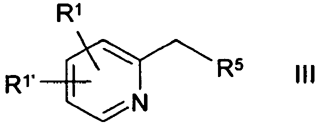 worin
   R¹, R^{1'} und R⁵ die in Anspruch 1 angegebenen Bedeutungen haben,
   und mit einer Verbindung der Formel IV 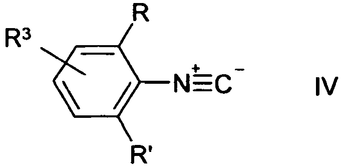 worin
   R, R' und R³ die in Anspruch 1 angegebenen Bedeutungen haben, umsetzt,
   und/oder
   eine Base oder Säure der Formel I in eines ihrer Salze umwandelt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Für alle Reste, die mehrfach auftreten, gilt, daß deren Bedeutungen unabhängig voneinander sind.
Vor- und nachstehend haben die Reste bzw. Parameter X, Y, R, R', R¹, R^{1'}, R², R^{2'}, R³, R⁴, R^{4'}, R⁵ und n die bei der Formel I angegebenen Bedeutungen, falls nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methyl-propyl, 1-Ethyl-2-methylpropyl, 1,1,2- oder 1,2,2-Trimethylpropyl, weiter bevorzugt z. B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.
Cycloalkyl bedeutet vorzugsweise Cyclopropyl, Cyclobutyl, Cylopentyl, Cyclohexyl oder Cycloheptyl.

R, R' bedeuten vorzugsweise, jeweils unabhängig voneinander A oder Hal, besonders bevorzugt Methyl, Ethyl, Propyl, Isopropyl oder Chlor.
R¹, R^{1'} bedeuten vorzugsweise H oder F.

R², R^{2'} bedeuten vorzugsweise, jeweils unabhängig voneinander H, A oder OA, besonders bevorzugt H, Methyl, Ethyl oder Methoxy.
R³ bedeutet vorzugsweise H.
R⁴, R^{4'} bedeuten vorzugsweise, jeweils unabhängig voneinander H oder CH₃.
R⁵ bedeutet vorzugsweise CN.

X bedeutet vorzugsweise O; Y fehlt vorzugsweise.
Hal bedeutet vorzugsweise F, Cl oder Br, aber auch I.
m bedeutet vorzugsweise 1 oder 2; n bedeutet vorzugsweise 0 oder 1, besonders bevorzugt 1.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ij ausgedrückt werden, die der Formel I entsprechen und worin die nicht näher bezeichneten Reste die bei der Formel angegebenen Bedeutung haben, worin jedoch
- in Ia: A unverzweigtes oder verzweigtes Alkyl mit 1, 2, 3, 4, 5 oder 6 C-Atomen, worin 1-5 H-Atome durch F ersetzt sein können,
bedeutet;
- in Ib: R, R' jeweils unabhängig voneinander A oder Hal bedeuten;
- in Ic: R¹, R^{1'} jeweils unabhängig voneinander H oder F bedeuten;
- in Id: R², R^{2'} jeweils unabhängig voneinander H, A oder OA bedeuten;
- in Ie: R⁴, R^{4'} jeweils unabhängig voneinander H oder CH₃ bedeuten;
- in If: R³ H bedeutet;
- in Ig: R⁵ CN bedeutet;
- in Ih: X O bedeutet;
- in Ii: Y fehlt
bedeutet;
- in Ij: R, R' jeweils unabhängig voneinander A oder Hal,
R¹, R^{1'} jeweils unabhängig voneinander H oder F,
R², R^{2'} jeweils unabhängig voneinander H, A oder OA,
R³ H,
R⁴, R^{4'} jeweils unabhängig voneinander H oder CH₃,
R⁵ CN,
X O,
Y fehlt,
- A: unverzweigtes oder verzweigtes Alkyl mit 1, 2, 3, 4, 5 oder 6 C-Atomen, worin 1-5 H-Atome durch F ersetzt sein können,
- Hal: F, Cl, Br oder I,
- n: 1
bedeuten;
sowie ihre pharmazeutisch verwendbaren Solvate, Salze und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Ausgangsverbindungen der Formeln II,III und IV sind in der Regel bekannt. Sind sie neu, so können sie aber nach an sich bekannten Methoden hergestellt werden.

Verbindungen der Formel I können vorzugsweise erhalten werden, indem man Verbindungen der Formel II mit Verbindungen der Formel III und IV umsetzt.

Die Umsetzung erfolgt in der Regel in einem inerten Lösungsmittel, in Gegenwart einer organischen Base, wie z.B. 1,8-Diazabicyclo[5.4.0]undec-7-en (DBU).

Die Reaktionszeit liegt je nach den angewendeten Bedingungen zwischen einigen Minuten und 14 Tagen, die Reaktionstemperatur zwischen etwa 0° und 150°, normalerweise zwischen 30° und 120°, besonders bevorzugt zwischen 60° und 110°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel; besonders bevorzugt ist n-Butanol.

Die Umsetzung wird in der Regel als Eintopfreaktion durchgeführt. Man kann die Reaktion aber auch zweistufig durchführen. Hierbei wird zunächst eine Verbindung der Formel II mit einer Verbindung der Formel III umgesetzt. Dabei entsteht ein Intermediat, das dann mit einer Verbindung der Formel IV umgesetzt wird.
Das Intermediat, das bei der Herstellung von "A1" auftritt, hat folgende Struktur:

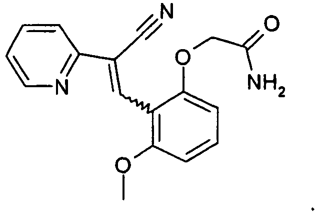

### Pharmazeutische Salze und andere Formen

Die genannten Verbindungen der Formel I lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, lodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der Verbindungen der Formel I Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der Formel I der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkyl-halogeniden, z.B. Benzylchlorid und Phenethylbromid, quarternisieren. Mit solchen Salzen können sowohl wasser- als auch öllösliche Verbindungen der Formel I hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von sauren Verbindungen der Formel I werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine Verbindung der Formel I mehr als eine Gruppe, die solche pharmazeutisch unbedenklichen Salze bilden kann, so umfaßt die Formel I auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Solvate, Salze und Stereoisomere, einschließlich deren Mischungen in allen Verhältnissen, sowie gegebenenfalls Träger- und/oder Hilfsstoffe.

Diese Zubereitungen können als Arzneimittel in der Human- oder Veterinärmedizin verwendet werden.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder H ilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die Wirkstoffe können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindungen enthält. Sirupe lassen sich herstellen, indem die Verbindungen in einer wäßrigen Lösung mit geeignetem Geschmack gelöst werden, während Elixiere unter Verwendung eines nichttoxischen alkoholischen Vehikels hergestellt werden. Suspensionen können durch Dispersion der Verbindungen in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze und Solvate, sowie die anderen Wirkstoffe lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze und Solvate, sowie die anderen Wirkstoffe können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insuftlatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I sowie des anderen Wirkstoffs hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer Verbindung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der Verbindung per se bestimmt werden.

Gegenstand der Erfindung ist ferner die Verwendung von Verbindungen der Formel I, in Kombination mit mindestens einem weiteren Arzneimittelwirkstoff, vorzugsweise zur Behandlung des Typ 1 und Typ 2 Diabetes, insbesondere zur Blutzuckersenkung.

Als weitere Wirkstoffe für die Kombinationspräparate sind geeignet:
Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart. Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glycogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT- 501, GI 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, AVE 0897 oder wie in WO 00/64888, WO 00/64876, WO 03/20269 beschrieben, verabreicht.

Bei einer Ausführungsform der Erfindung werden die Verbindungen der Formel I in Kombination mit einem Fihrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht. Bei einer Ausführungsform der Erfindung werden die Verbindungen der Formel I in Kombination mit Gallensäureresorptionsinhibitor (siehe z.B. US 6,245,744 oder US 6,221, 897), wie z.B. HMR 1741, verabreicht.

Bei einer Ausführungsform der Erfindung werden die Verbindungen der Formel I in Kombination mit einem CETP-Inhibitor, wie z.B. JTT-705, verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht. Bei einer Ausführungsform der Erfindung werden die Verbindungen der Formel I in Kombination mit einem Lipoprotein(a) antagonist, wie z.B. CI-1027 oder Nicotinsäure, verabreicht. Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Biguanid, wie z.B. Metformin, verabreicht.

Bei einer anderen Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamineregulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl)-cyclohexylmethyl}-amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/ 63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B.1-(4-Chloro-3-methanesulfonylmethyl-phenyl)-2-[2-(2,3-dimethyl-1H-indol-6-yloxy)ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 10 00/71549), 5HT-Agonisten z.B. 1-(3-Ethyl-benzofuran-7-yl)-piperazin Oxalsäuresalz (WO 01/09111), Bombesin-Agonisten, Galanin- Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isochinolin-2-carbonsäure-tert-butylester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity, Drugs of the Future (2001), 26(9), 873-881), DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez Ambrosi, Javier; Fruhbeck, Gema, Expert Opinion on Pharmacotherapy (2001), 2(10), 1615-1622.

Bei einer Ausführungsform ist der weitere Wirkstoff Dexamphatamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties & Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, daß jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

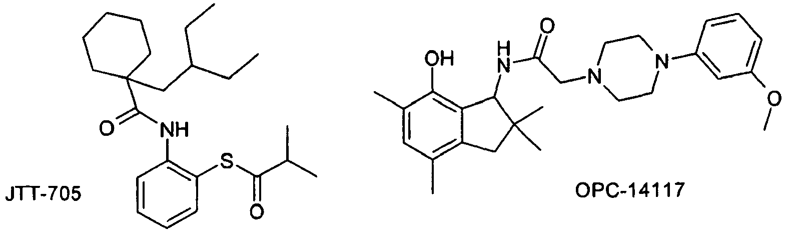

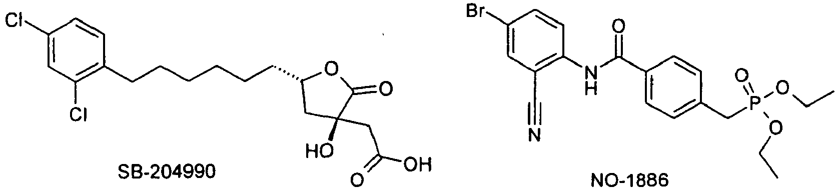

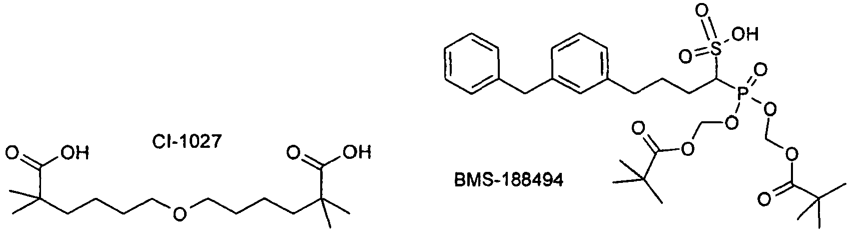

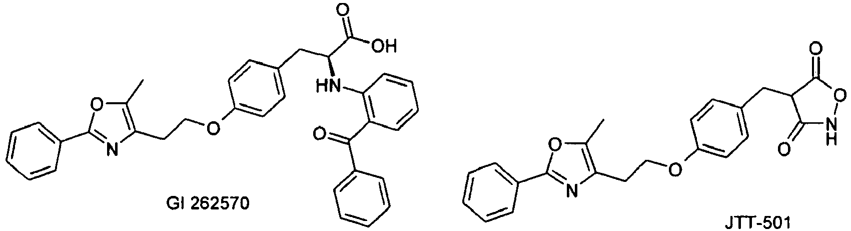

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Derivate, Solvate, Salze und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Solvate und Stereoisomere, einschließlich deren Mischungen in allen Verhältnissen,
und einer wirksamen Menge eines weiteren Arzneimittelwirkstoffs gelöst oder in lyophilisierter Form vorliegt.

Die Verbindungen können auf ihre SGLT-Hemmeigenschaften mittels BHK-Zellen getestet werden, die SGLT1 und SGLT2 exprimieren. Die Herstellung der Zellen und die Untersuchung kann wie nachstehend beschrieben durchgeführt werden.

### Konstruktion und Expression von SGLT1 in BHK-Zellen

Zur Konstruktion des SGLT1-Expressionsvektors (KL225) wurde das SLC5A1-Gen (homolog zu NM_000343) aus einer cDNA-Bank mittels Standard-PCR-Technologie amplifiziert und über Nhel/Xhol-Stellen in den pcDNA3.1-Expressionsvektor (Invitrogen) kloniert, welcher Neomycin als Selektionsmarker enthielt. Bei diesem Vektor verwendet die Transkription den Enhancer/Promotor des Cytomegalievirus beim Menschen.

Der fertige Vektor KL225 wurde zusammen mit einem zusätzlichen Vektor, der ein Dihydrofolatreduktase-Gen als Selektionsmarker enthielt, in Zellen eingebracht. Die Transfektion in BHK21-Zellen (ATCC CCL-10), gezüchtet in DMEM-Medium (GIBCO/ BRL), angereichert mit 10% fötalem Kälberserum (FCS) und 20 mM Glutamin, erfolgte mit Calciumphosphat-Transfektionen gemäß Graham, F.L. und van der Ebb, A.J. (1973), Virology 52: 456 mit 5 - 20 µg ungeschnittenen Plasmiden für 10⁷ Zellen. Stabile Transfektanten wurden in Medium selektiert, das 1 mg/ml G418 (GIBCO/BRL) und 20 - 5000 nM Methotrexat als Endkonzentration enthielt, wobei nur Zellen, die das Neomycin-Gen exprimierten und das dhfr-Gen überexprimierten, wachsen konnten. Nach 2 - 3 Wochen Wachstum wurden die Zellen kloniert (0,5 Zellen/Vertiefung) und die Klone in Radioaktivitäts-Aufnahme-Tests auf SGLT-Expression untersucht.

### Konstruktion und Expression von SGLT2 in BHK-Zellen

Zur Konstruktion des SGLT2-Expressionsvektors (KL224) wurde das SLC5A2-Gen (homolog zu NM_003041) aus einer cDNA-Bank mittels Standard-PCR-Technologie amplifiziert und über NheI/XhoI-Stellen in PCIneo Expressionsvektor (Promega) kloniert, der Neomycin als Selektionsmarker enthielt. In diesem Vektor verwendet die Transkription den Enhancer/Promotor des Cytomegalie-Virus beim Menschen und das Polyadenylierungssignal von SV40.
Der fertige Vektor KL224 wurde zusammen mit einem zusätzlichen Vektor, der ein Dihydrofolatreduktase-Gen als Selektionsmarker enthielt, in Zellen eingebracht. Die Transfektion in BHK21-Zellen (ATCC CCL-10), gezüchtet in DMEM-Medium (GIBCO/ BRL), angereichert mit 10% fötalem Kälberserum (FCS) und 20 mM Glutamin, erfolgte mit Calciumphosphat-Transfektionen gemäß Graham, F. L. und van der Ebb, A.J. (1973), Virology 52: 456 mit 5 - 20 µg ungeschnittenen Plasmiden für 10⁷ Zellen. Stabile Transfektanten wurden in Medium selektiert, das 1 mg/ml G418 (GIBCO/BRL) und 20 - 5000 nM Methotrexat als Endkonzentration enthielt, wobei nur Zellen, die das Neomycin-Gen exprimierten und das dhfr-Gen überexprimierten, wachsen konnten. Nach 2 - 3 Wochen Wachstum wurden die Zellen kloniert (0,5 Zellen/Vertiefung) und die Klone in Radioaktivitäts-Aufnahme-Tests auf SGLT-Expression untersucht.

### Verfahren zur Messung der SGLT1/2-Aktivität

Prinzipiell wurde die Aufnahme von ¹⁴C-α-Methyl-D-glucopyranosid (AMG) beispielsweise in Xenopus-Oozyten, denen die entsprechende cRNA injiziert wurde, beschrieben (beispielsweise Wen-Sen Lee et al. (1994), J. Biol. Chem. 269, 12032-12039; Guofeng You et al. (1995), J. Biol. Chem. 270, 29365-29371).
Ein in 96 Vertiefungen durchgeführter Test auf Zellbasis wurde entwickelt und an die HTS-Anforderungen angepasst:
BHK-Zellen (transfiziert mit SGLT1 oder SGLT2) wurden in Mikrotiterplatten mit 96 Vertiefungen (Cultureplates, Perkin Eimer) überimpft. Nach mindestens 24 Std. wurde das Medium enternt und die Zellschicht wurde mit Testpuffer (140 mM NaCl, 2 mM KCI, 1 mM CaCl₂, 1 mM MgCl₂, 10 mM HEPES, 5 mM Tris, mit 1 M KOH auf pH-Wert 7,4 eingestellt) gewaschen. Nach Zugabe von 40 µl Testpuffer 50 µl AMG (50 µM für SGLT1 bzw. 2 mM für SGLT2) in Gegenwart oder Abwesenheit von Verbindungen wurden die Zellen in einem Gesamtvolumen von 100 µl bei 37°C für 90 min. inkubiert. Der Überstand wurde abgesaugt und verworfen. Die Zellen wurden gewaschen und durch Zugabe von 50 µl Wasser lysiert. Nach 10 min bei Raumtemperatur wurden 200 µl Micrsoscint 40 (Perkin Elmer) hinzu gefügt. Die Radioaktivität wurde in einem Topcount Mikroplatten-Szintillationszähler (Perkin Elmer) gezählt. Die unspezifische Aufnahme wurde in natriumfreiem Testpuffer (266 mM Saccharose, 2 mM KCI, 1 mM CaCl₂, 1 mM MgCl₂, 10 mM HEPES, 5 mM Tris, mit 1 M KOH auf pH-Wert 7,4 eingestellt) bestimmt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
Massenspektrometrie (MS): EI (Elektronenstoß-Ionisation) M⁺
FAB (Fast Atom Bombardment) (M+H)⁺
ESI (Electrospray lonization) (M+H)⁺ (wenn nichts anderes angegeben)

### Beispiel 1

Die Herstellung von 2-{2-[1-Cyan-3-(2,6-dimethyl-phenylamino)-indolizin-2-yl]-3-methoxy-phenoxy}-acetamid ("A1") erfolgt analog nachstehendem Schema:

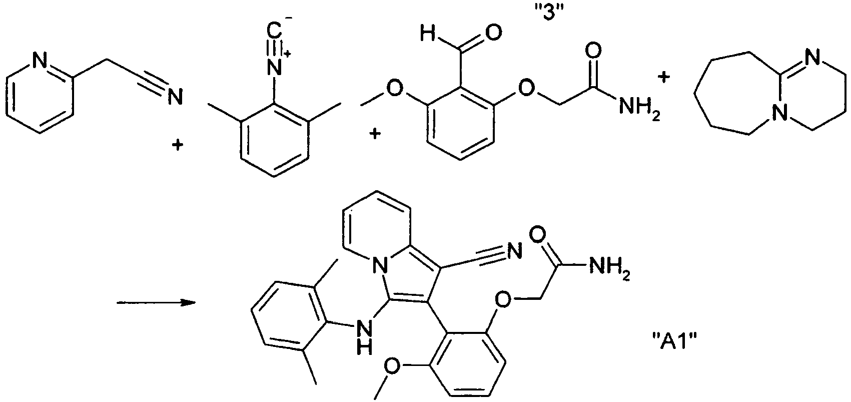

0,12 g (1.0 mmol) 2-Pyridylacetonitril, 0,14g (1.0mmol) 2,6-Dimethylphenylisocyanid und 0,21g (1.0mmol) **"3"** und werden in 15mL 1-Butanol gelöst und anschließend 15µL (1.4mmol) 1,8-Diazabicyclo[5.4.0]undec-7-en hinzugefügt. Das Reaktionsgemisch wird auf 100°C erhitzt und 20 h bei dieser Temperatur gerührt. Nach dem Abkühlen wird anschließend wie üblich aufgearbeitet und man erhält so 0,07 g (15%) "A1" als Feststoff; F. 227-229°; MS-EI (M⁺) = 440; ¹H-NMR (DMSO-d₆) δ 7,87 (1H, d, J = 7,0 Hz), 7,60 (1H, d, J = 8,9 Hz), 7,45 (1H, s), 7,22 (1 H, t, J = 8,4 Hz), 7,10 (1 H, dd, J = 6,7 und 8,1 Hz), 6,96 (1 H, s), 6,88-6,84 (2H, m), 6,75 (2H, d, J = 7,1 Hz), 6,65-6,60 (2H, m), 6,47 (1H, d, J = 8,4 Hz), 4,38 (2H, dd, J = 15,1 und 11,2 Hz), 3,65 (3H, s), 1,80 (6H, s).

Analog erhält man die nachstehenden Verbindungen

| Nr. | Name und/oder Struktur | F. [°C] / MS-EI (M⁺) |
|---|---|---|
| "A2" | 2-{2-[1-Cyan-3-(2,6-dimethyl-phenylamino)-indolizin-2-yl]-phenoxy}-acetamid | 193-195 / 410 |
| ¹H-NMR (DMSO-d₆) δ 7,83 (1H, d, J = 7,0 Hz), 7,66 (1H, d, J = 8,9 Hz), 7,51 (1H, s), 7,32-7,25 (1H, m), 7,18-7,10 (1H, m), 7,09 (1H, s), 6,98 (1H, t, J = 7,4), 6,90 (2H, t, J = 7,9 Hz), 6,78 (2H, d, J = 7,4 Hz), 6,64 (1H, t, J = 7,4 Hz), 4,49 (2H, s), 1,80 (6H, s) | | |
| "A3" | 2-{2-[1-Cyan-3-(2,6-dimethyl-phenylamino)-indolizin-2-yl]-5-methoxy-3-methylphenoxy}-acetamid | 226-227 / 454 |
| ¹H-NMR (DMSO-d₆) δ 7,88 (1H, d, J = 7,0 Hz), 7,68 (1H, d, J = 8,9 Hz), 7,44 (1H, s), 7,13 (1H, dd, J = 6,8 Hz), 7,00 (1H, s), 6,93 (1H, s), 6,88 (1H, t, J = 6,8 Hz), 6,81-6,76 (2H, m), 6,66 (1H, t, J 0 7,4 Hz), 6,46 (1H, d, J = 2,0 Hz), 6,21 (1H, d, J = 2,1 Hz), 4,33 (2H, s), 3,72 (3H, s), 2,11 (3H, s), 1,80 (6H, s) | | |
| "A4" | 2-{2-[1-Cyan-3-(2,6-dimethyl-phenylamino)-6-fluor-indolizin-2-yl]-phenoxy}-acetamid | |
| | 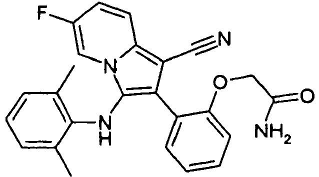 | |
| "A5" | 2-{2-[1-Cyan-3-(2,6-dichlor-phenylamino)-indolizin-2-yl]-phenoxy}-acetamid | |
| "A6" | 2-{2-[1-Cyan-3-(2-chlor-6-methyl-phenylamino)-indolizin-2-yl]-phenoxy}-acetamid | |
| "A7" | 2-{2-[1-Cyan-3-(2-ethyl-6-methyl-phenylamino)-indolizin-2-yl]-phenoxy}-acetamid | |
| "A8" | 2-{2-[1-Cyan-3-(2,6-dimethyl-phenylamino)-6,8-difluor-indolizin-2-yl]-phenoxy}-acetamid | |
| "A9" | 2-{2-[1-Cyan-3-(2,6-dimethyl-phenylamino)-6-fluor-indolizin-2-yl]-3-methyl-phenoxy}-acetamid | |
| "A10" | 2-{2-[1-Cyan-3-(2,6-dimethyl-phenylamino)-6-fluor-indolizin-2-yl]-3-methoxy-phenoxy}-acetamid | |
| "A11" | 2-{2-[1-Cyan-3-(2,6-dimethyl-phenylamino)-indolizin-2-yl]-3,5-dimethoxy-phenoxy)-acetamid | |
| "A12" | 2-{2-[1-Cyan-3-(2,6-dimethyl-phenylamino)-indolizin-2-yl]-3-ethyl-phenoxy}-acetamid | 236-237 |
| "A13" | 2-{2-[1-Cyan-3-(2,6-dimethyl-phenylamino)-6-fluor-indolizin-2-yl)-5-methoxy-phenoxy}-acetamid | |
| "A14" | 2-{2-[1-Cyan-3-(2,6-dimethyl-phenylamino)-6,8-difluor-indolizin-2-yl]-3-methoxy-phenoxy}-acetamid | |
| "A15" | 2-{2-[1-Cyan-3-(2,6-dimethyl-phenylamino)-6,8-difluor-indolizin-2-yl]-3-ethyl-phenoxy)-acetamid | |
| "A16" | 2-{2-[1-Cyan-3-(2,6-dimethyl-phenylamino)-6-fluor-indolizin-2-yl]-3-ethyl-phenoxy}-acetamid | |
| "A17" | 2-{2-[1-Cyan-3-(2,6-dimethyl-phenylamino)-6-fluor-indolizin-2-yl]-3,5-dimethoxy-phenoxy}-acetamid | |
| "A18" | 2-{2-[1-Cyan-3-(2,6-dichloro-phenylamino)-indolizin-2-yl]-3-ethyl-phenoxy}acetamid | 224-226 |

### Pharmakologische Daten

Affinität zu Rezeptoren

**Tabelle 1**

| Verbindung Nr. | SGLT₁-IC₅₀ | SGLT₂-IC₅₀ |
|---|---|---|
| "A1" | B | A |
| "A2" | B | A |
| "A3" | | |

| | | |
|---|---|---|
| IC₅₀: 10 nM - 1 µM = A 1 µM - 10 µM =B > 10 µM =C | | |

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 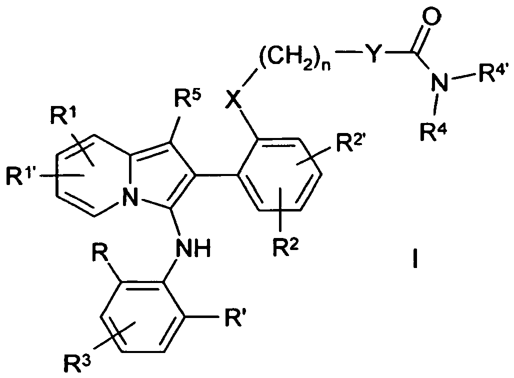 worin
R, R' jeweils unabhängig voneinander A, OA oder Hal,
R¹, R^{1'} jeweils unabhängig voneinander H, A, F oder Cl,
R², R^{2'} jeweils unabhängig voneinander H, Hal, A, OH, OA, CN, NO₂, NR⁴ R^{4'}, CH₂NR⁴R^{4'}, O(CH₂)ₘNR⁴R^{4'}, O(CH₂)ₘOR⁴, NH(CH₂)ₘNR⁴R^{4'}, O(C=O)(CH₂)ₘNR⁴R^{4'}, NH(C=O)(CH₂)ₘNR⁴R^{4'}, CH₂O(CH₂)ₘNR⁴R^{4'}, CH₂OR⁴ oder COOR⁴,
R³ H, A oder Hal,
R⁴, R^{4'} jeweils unabhängig voneinander H oder A,
R⁵ CN, COOA oder COOH,
X, Y jeweils unabhängig voneinander O, NH, CH₂ oder fehlt,
A unverzweigtes oder verzweigtes Alkyl mit 1-10 C-Atomen, worin 1-7 H-Atome durch F ersetzt sein können, oder Cycloalkyl mit 3-7 C-Atomen,
Hal F, Cl, Br oder I,
m 1, 2 oder 3,
n 0, 1 oder 2,
bedeuten, sowie ihre pharmazeutisch verwendbaren Solvate, Salze und Stereoisomere, einschließlich deren Mischungen in allen Verhältnissen.

2. Verbindungen nach Anspruch 1, worin
A unverzweigtes oder verzweigtes Alkyl mit 1, 2, 3, 4, 5 oder 6 C-Atomen, worin 1-5 H-Atome durch F ersetzt sein können,
bedeutet,
sowie ihre pharmazeutisch verwendbaren Solvate, Salze und Stereoisomere, einschließlich deren Mischungen in allen Verhältnissen.

3. Verbindungen nach Anspruch 1 oder 2, worin
R, R' jeweils unabhängig voneinander A oder Hal
bedeuten,
sowie ihre pharmazeutisch verwendbaren Solvate, Salze und Stereoisomere, einschließlich deren Mischungen in allen Verhältnissen.

4. Verbindungen gemäß einem oder mehreren der Ansprüche 1-3, worin
R¹, R^{1'} jeweils unabhängig voneinander H oder F bedeuten,
sowie ihre pharmazeutisch verwendbaren Solvate, Salze und Stereoisomere, einschließlich deren Mischungen in allen Verhältnissen.

5. Verbindungen gemäß einem oder mehreren der Ansprüche 1-4, worin
R², R^{2'} jeweils unabhängig voneinander H, A oder OA
bedeuten,
sowie ihre pharmazeutisch verwendbaren Solvate, Salze und Stereoisomere, einschließlich deren Mischungen in allen Verhältnissen.

6. Verbindungen gemäß einem oder mehreren der Ansprüche 1-5, worin
R⁴, R^{4'} jeweils unabhängig voneinander H oder CH₃ bedeuten,
sowie ihre pharmazeutisch verwendbaren Solvate, Salze und Stereoisomere, einschließlich deren Mischungen in allen Verhältnissen.

7. Verbindungen gemäß einem oder mehreren der Ansprüche 1-6, worin
R³ H bedeutet,
sowie ihre pharmazeutisch verwendbaren Solvate, Salze und Stereoisomere, einschließlich deren Mischungen in allen Verhältnissen.

8. Verbindungen gemäß einem oder mehreren der Ansprüche 1-6, worin
R⁵ CN bedeutet,
sowie ihre pharmazeutisch verwendbaren Solvate, Salze und Stereoisomere, einschließlich deren Mischungen in allen Verhältnissen.

9. Verbindungen gemäß einem oder mehreren der Ansprüche 1-8, worin
X O bedeutet,
sowie ihre pharmazeutisch verwendbaren Solvate, Salze und Stereoisomere, einschließlich deren Mischungen in allen Verhältnissen.

10. Verbindungen gemäß einem oder mehreren der Ansprüche 1-9, worin
Y fehlt
bedeutet,
sowie ihre pharmazeutisch verwendbaren Solvate, Salze und Stereoisomere, einschließlich deren Mischungen in allen Verhältnissen.

11. Verbindungen gemäß einem oder mehreren der Ansprüche 1-10,
worin
R, R' jeweils unabhängig voneinander A oder Hal,
R¹, R^{1'} jeweils unabhängig voneinander H oder F,
R², R^{2'} jeweils unabhängig voneinander H, A oder OA,
R³ H,
R⁴, R^{4'} jeweils unabhängig voneinander H oder CH₃,
R⁵ CN,
X O,
Y fehlt,
A unverzweigtes oder verzweigtes Alkyl mit 1, 2, 3, 4, 5 oder 6 C-Atomen, worin 1-5 H-Atome durch F ersetzt sein können,
Hal F, Cl, Br oder I,
n 1
bedeuten,
sowie ihre pharmazeutisch verwendbaren Solvate, Salze und Stereoisomere, einschließlich deren Mischungen in allen Verhältnissen.

12. Verbindungen nach Anspruch 1 ausgewählt aus der Gruppe
| Nr. | Name |
|---|---|
| "A1" | 2-{2-[1-Cyan-3-(2,6-dimethyl-phenylamino)-indolizin-2-yl]-3-methoxy-phenoxy}-acetamid |
| "A2" | 2-{2-[1-Cyan-3-(2,6-dimethyl-phenylamino)-indolizin-2-yl]-phenoxy}-acetamid |
| "A3" | 2-{2-[1-Cyan-3-(2,6-dimethyl-phenylamino)-indolizin-2-yl]-5-methoxy-3-methylphenoxy}-acetamid |
| "A4" | 2-{2-[1-Cyan-3-(2,6-dimethyl-phenylamino)-6-fluor-indolizin-2-yl]-phenoxy}-acetamid |
| "A5" | 2-{2-[1-Cyan-3-(2,6-dichlor-phenylamino)-indolizin-2-yl]-phenoxy}-acetamid |
| "A6" | 2-{2-[1-Cyan-3-(2-chlor-6-methyl-phenylamino)-indolizin-2-yl]-phenoxy}-acetamid |
| "A7" | 2-{2-[1-Cyan-3-(2-ethyl-6-methyl-phenylamino)-indolizin-2-yl]-phenoxy}-acetamid |
| "A8" | 2-{2-[1-Cyan-3-(2,6-dimethyl-phenylamino)-6,8-difluor-indolizin-2-yl]-phenoxy}-acetamid |
| "A9" | 2-{2-[1-Cyan-3-(2,6-dimethyl-phenylamino)-6-fluor-indolizin-2-yl]-3-methyl-phenoxy}-acetamid |
| "A10" | 2-{2-[1-Cyan-3-(2,6-dimethyl-phenylamino)-6-fluor-indolizin-2-yl]-3-methoxy-phenoxy}-acetamid |
| "A11" | 2-{2-[1-Cyan-3-(2,6-dimethyl-phenylamino)-indolizin-2-yl]-3,5-dimethoxy-phenoxy}-acetamid |
| "A12" | 2-{2-[1-Cyan-3-(2,6-dimethyl-phenylamino)-indolizin-2-yl]-3-ethyl-phenoxy}-acetamid |
| "A13" | 2-{2-[1-Cyan-3-(2,6-dimethyl-phenylamino)-6-fluor-indolizin-2-yl]-5-methoxy-phenoxy}-acetamid |
| "A14" | 2-{2-[1-Cyan-3-(2,6-dimethyl-phenylamino)-6,8-difluor-indolizin-2-yl]-3-methoxy-phenoxy}-acetamid |
| "A15" | 2-{2-[1-Cyan-3-(2,6-dimethyl-phenylamino)-6,8-difluor-indolizin-2-yl]-3-ethyl-phenoxy}-acetamid |
| "A16" | 2-{2-[1-Cyan-3-(2,6-dimethyl-phenylamino)-6-fluor-indolizin-2-yl]-3-ethyl-phenoxy}-acetamid |
| "A17" | 2-{2-[1-Cyan-3-(2,6-dimethyl-phenylamino)-6-fluor-indolizin-2-yl]-3,5-dimethoxy-phenoxy}-acetamid |
sowie ihre pharmazeutisch verwendbaren Solvate, Salze und Stereoisomere, einschließlich deren Mischungen in allen Verhältnissen.

13. Verfahren zur Herstellung von Verbindungen der Formel I nach den Ansprüchen 1-12 sowie ihrer pharmazeutisch verwendbaren Solvate, Salze und Stereoisomere, **dadurch gekennzeichnet, daß** man
a) eine Verbindung der Formel II 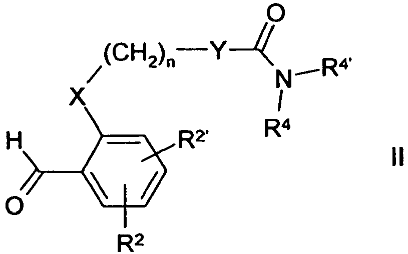 worin
X, Y, R², R^{2'}, R⁴, R^{4'} and n die in Anspruch 1 angegebenen Bedeutungen haben,
mit einer Verbindung der Formel III 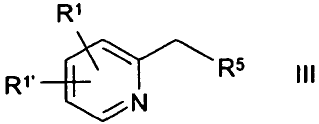 worin
R¹, R^{1'} und R⁵ die in Anspruch 1 angegebenen Bedeutungen haben, und mit einer Verbindung der Formel IV 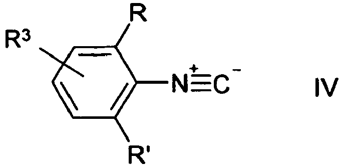 worin R, R' und R³ die in Anspruch 1 angegebenen Bedeutungen haben,
umsetzt,
und/oder
eine Base oder Säure der Formel I in eines ihrer Salze umwandelt.

14. Arzneimittel, enthaltend mindestens eine Verbindung der Formel I nach einem oder mehreren der Ansprüche 1 bis 12 und/oder ihre pharmazeutisch verwendbaren Solvate, Salze und Stereoisomere, einschließlich deren Mischungen in allen Verhältnissen, sowie gegebenenfalls Träger- und/oder Hilfsstoffe.

15. Arzneimittel enthaltend mindestens eine Verbindung der Formel I gemäß einem oder mehreren der Ansprüche 1 bis 12 und/oder ihre pharmazeutisch verwendbaren Solvate und Stereoisomere, einschließlich deren Mischungen in allen Verhältnissen, und mindestens einen weiteren Arzneimittelwirkstoff.

16. Verwendung von Verbindungen gemäß einem oder mehreren der Ansprüche 1 bis 12 und/oder ihre physiologisch unbedenklichen Salze, Salze und Solvate zur Herstellung eines Arzneimittels zur Behandlung des Typ 1 und Typ 2 Diabetes.

17. Verwendung von Verbindungen gemäß einem oder mehreren der Ansprüche 1 bis 12 und/oder ihre physiologisch unbedenklichen Salze, Salze und Solvate zur Herstellung eines Arzneimittels zur Blutzuckersenkung.

18. Verwendung von Verbindungen gemäß einem oder mehreren der Ansprüche 1 bis 12 und/oder ihre physiologisch unbedenklichen Salze, Salze und Solvate und einem weiteren Arzneimittelwirkstoff zur Herstellung eines Arzneimittels zur Behandlung des Typ 1 und Typ 2 Diabetes.

19. Set (Kit), bestehend aus getrennten Packungen von
(a) einer wirksamen Menge an einer Verbindung der Formel I gemäß einem oder mehreren der Ansprüche 1 bis 12 und/oder ihrer pharmazeutisch verwendbaren Solvate, Salze und Stereoisomere, einschließlich deren Mischungen in allen Verhältnissen, und
(b) einer wirksamen Menge eines weiteren Arzneimittelswirkstoffs.

20. Verwendung von Verbindungen gemäß einem oder mehreren der Ansprüche 1 bis 12 und/oder ihre physiologisch unbedenklichen Salze, Salze und Solvate und einem weiteren Arzneimittetwirkstoff zur Herstellung eines Arzneimittels zur Blutzuckersenkung.

## Claims

1. Compounds of the formula I 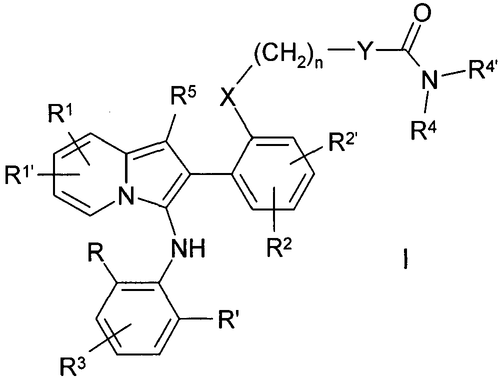 in which
R, R' each, independently of one another, denote A, OA or Hal,
R¹, R^{1'} each, independently of one another, denote H, A, F or Cl,
R², R^{2'} each, independently of one another, denote H, Hal, A, OH, OA, CN, NO₂, NR⁴R^{4'}, CH₂NR⁴R^{4'}, O(CH₂)ₘNR⁴R^{4'}, O(CH₂)ₘOR⁴, NH(CH₂)ₘNR⁴R^{4'}, O(C=O)(CH₂)ₘNR⁴R^{4'}, NH(C=O)(CH₂)ₘNR⁴R^{4'}, CH₂O(CH₂)ₘNR₄R^{4'}, CH₂OR⁴ or COOR⁴,
R³ denotes H, A or Hal,
R⁴, R^{4'} each, independently of one another, denote H or A,
R⁵ denotes CN, COOA or COOH,
X, Y each, independently of one another, denote O, NH, CH₂ or are absent,
A denotes unbranched or branched alkyl having 1-10 C atoms, in which 1-7 H atoms may be replaced by F, or cycloalkyl having 3-7 C atoms,
Hal denotes F, Cl, Br or I,
m denotes 1, 2 or 3,
n denotes 0, 1 or 2,
and pharmaceutically usable solvates, salts and stereoisomers thereof, including mixtures thereof in all ratios.

2. Compounds according to Claim 1 in which
A denotes unbranched or branched alkyl having 1, 2, 3, 4, 5 or 6 C atoms, in which 1-5 H atoms may be replaced by F,
and pharmaceutically usable solvates, salts and stereoisomers thereof, including mixtures thereof in all ratios.

3. Compounds according to Claim 1 or 2 in which
R, R' each, independently of one another, denote A or Hal,
and pharmaceutically usable solvates, salts and stereoisomers thereof, including mixtures thereof in all ratios.

4. Compounds according to one or more of Claims 1-3 in which
R¹, R^{1'} each, independently of one another, denote H or F,
and pharmaceutically usable solvates, salts and stereoisomers thereof, including mixtures thereof in all ratios.

5. Compounds according to one or more of Claims 1-4 in which
R², R^{2'} each, independently of one another, denote H, A or OA,
and pharmaceutically usable solvates, salts and stereoisomers thereof, including mixtures thereof in all ratios.

6. Compounds according to one or more of Claims 1-5 in which
R⁴, R^{4'} each, independently of one another, denote H or CH₃,
and pharmaceutically usable solvates, salts and stereoisomers thereof, including mixtures thereof in all ratios.

7. Compounds according to one or more of Claims 1-6 in which
R³ denotes H,
and pharmaceutically usable solvates, salts and stereoisomers thereof, including mixtures thereof in all ratios.

8. Compounds according to one or more of Claims 1-6 in which
R⁵ denotes CN,
and pharmaceutically usable solvates, salts and stereoisomers thereof, including mixtures thereof in all ratios.

9. Compounds according to one or more of Claims 1-8 in which
X denotes O,
and pharmaceutically usable solvates, salts and stereoisomers thereof, including mixtures thereof in all ratios.

10. Compounds according to one or more of Claims 1-9 in which
Y is absent,
and pharmaceutically usable solvates, salts and stereoisomers thereof, including mixtures thereof in all ratios.

11. Compounds according to one or more of Claims 1-10 in which
R, R' each, independently of one another, denote A or Hal,
R¹, R^{1'} each, independently of one another, denote H or F,
R², R^{2'} each, independently of one another, denote H, A or OA,
R³ denotes H,
R⁴, R^{4'} each, independently of one another, denote H or CH₃,
R⁵ denotes CN,
X denotes O,
Y is absent,
A denotes unbranched or branched alkyl having 1, 2, 3, 4, 5 or 6 C atoms, in which 1-5 H atoms may be replaced by F,
Hal denotes F, Cl, Br or I,
n denotes 1,
and pharmaceutically usable solvates, salts and stereoisomers thereof, including mixtures thereof in all ratios.

12. Compounds according to Claim 1 selected from the group
| No. | Name |
|---|---|
| "A1" | 2-{2-[1-Cyano-3-(2,6-dimethylphenylamino)indolizin-2-yl]-3-methoxyphenoxy}acetamide |
| "A2" | 2-{2-[1-Cyano-3-(2,6-dimethylphenylamino)indolizin-2-yl]phenoxy}acetamide |
| "A3" | 2-{2-[1-Cyano-3-(2,6-dimethylphenylamino)indolizin-2-yl]-5-methoxy-3-methylphenoxy}acetamide |
| "A4" | 2-{2-[1-Cyano-3-(2,6-dimethylphenylamino)-6-fluoro-indolizin-2-yl]phenoxy}acetamide |
| "A5" | 2-{2-[1-Cyano-3-(2,6-dichlorophenylamino)indolizin-2-yl]phenoxy}acetamide |
| "A6" | 2-{2-[1-Cyano-3-(2-chloro-6-methylphenylamino)-indolizin-2-yl]phenoxy}acetamide |
| "A7" | 2-{2-[1-Cyano-3-(2-ethyl-6-methylphenylamino)indolizin-2-yl]phenoxy)acetamide |
| "A8" | 2-{2-[1-Cyano-3-(2,6-dimethylphenylamino)-6,8-difluoro-indolizin-2-yl]phenoxy}acetamide |
| "A9" | 2-{2-[1-Cyano-3-(2,6-dimethylphenylamino)-6-fluoro-indolizin-2-yl]-3-methylphenoxy}acetamide |
| "A10" | 2-{2-[1-Cyano-3-(2,6-dimethylphenylamino)-6-fluoro-indolizin-2-yl]-3-methoxyphenoxy}acetamide |
| "A11" | 2-{2-[1-Cyano-3-(2,6-dimethylphenylamino)indolizin-2-yl]-3,5-dimethoxyphenoxy}acetamide |
| "A12" | 2-{2-[1-Cyano-3-(2,6-dimethylphenylamino)indolizin-2-yl]-3-ethylphenoxy}acetamide |
| "A13" | 2-{2-[1-Cyano-3-(2,6-dimethylphenylamino)-6-fluoro-indolizin-2-yl]-5-methoxyphenoxy}acetamide |
| "A14" | 2-{2-[1-Cyano-3-(2,6-dimethylphenylamino)-6,8-difluoro-indolizin-2-yl]-3-methoxyphenoxy}acetamide |
| "A15" | 2-{2-[1-Cyano-3-(2,6-dimethylphenylamino)-6,8-difluoro-indolizin-2-yl]-3-ethylphenoxy}acetamide |
| "A16" | 2-{2-[1-Cyano-3-(2,6-dimethylphenylamino)-6-fluoro-indolizin-2-yl]-3-ethylphenoxy}acetamide |
| "A17" | 2-{2-[1-Cyano-3-(2,6-dimethylphenylamino)-6-fluoro-indolizin-2-yl]-3,5-dimethoxyphenoxy}acetamide |
and pharmaceutically usable solvates, salts and stereoisomers thereof, including mixtures thereof in all ratios.

13. Process for the preparation of compounds of the formula I according to Claims 1-12 and pharmaceutically usable solvates, salts and stereoisomers thereof, **characterised in that**
a) a compound of the formula II 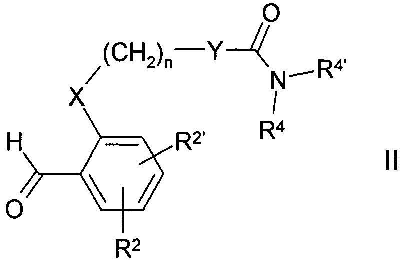 in which
X, Y, R², R^{2'}, R⁴, R^{4'} and n have the meanings indicated in Claim 1, is reacted with a compound of the formula III 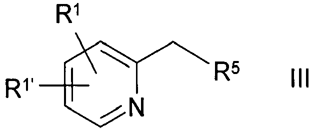 in which
R¹, R^{1'} and R⁵ have the meanings indicated in Claim 1,
and with a compound of the formula IV 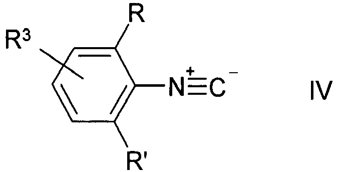 in which R, R' and R³ have the meanings indicated in Claim 1,
and/or
a base or acid of the formula I is converted into one of its salts.

14. Medicaments comprising at least one compound of the formula I according to one or more of Claims 1 to 12 and/or pharmaceutically usable solvates, salts and stereoisomers thereof, including mixtures thereof in all ratios, and optionally excipients and/or adjuvants.

15. Medicaments comprising at least one compound of the formula I according to one or more of Claims 1 to 12 and/or pharmaceutically usable solvates and stereoisomers thereof, including mixtures thereof in all ratios, and at least one further medicament active compound.

16. Use of compounds according to one or more of Claims 1 to 12 and/or physiologically acceptable salts and solvates thereof for the preparation of a medicament for the treatment of type 1 and type 2 diabetes.

17. Use of compounds according to one or more of Claims 1 to 12 and/or physiologically acceptable salts and solvates thereof for the preparation of a medicament for lowering blood sugar.

18. Use of compounds according to one or more of Claims 1 to 12 and/or physiologically acceptable salts and solvates thereof and a further medicament active compound for the preparation of a medicament for the treatment of type 1 and type 2 diabetes.

19. Set (kit) consisting of separate packs of
(a) an effective amount of a compound of the formula I according to one or more of Claims 1 to 12 and/or pharmaceutically usable solvates, salts and stereoisomers thereof, including mixtures thereof in all ratios,
and
(b) an effective amount of a further medicament active compound.

20. Use of compounds according to one or more of Claims 1 to 12 and/or physiologically acceptable salts and solvates thereof and a further medicament active compound for the preparation of a medicament for lowering blood sugar.

## Revendications

1. Composés de formule I 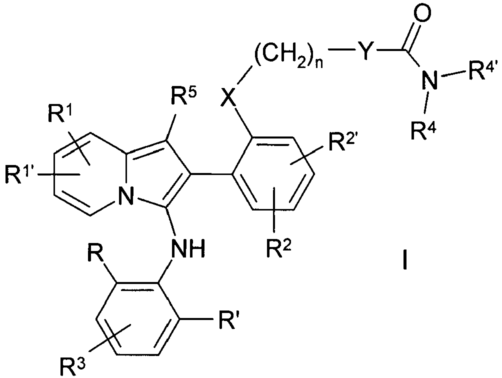 dans laquelle
R, R' désignent chacun, indépendamment l'un de l'autre, A, OA ou Hal,
R¹, R^{1'} désignent chacun, indépendamment l'un de l'autre, H, A, F ou Cl,
R², R^{2'} désignent chacun, indépendamment l'un de l'autre, H, Hal, A, OH, OA, CN, NO₂, NR⁴R^{4'}, CH₂NR⁴R^{4'}, O(CH₂)ₘNR⁴R^{4'}, O(CH₂)ₘOR⁴, NH(CH₂)ₘNR⁴R^{4'}, O(C=O)(CH₂)ₘNR⁴R^{4'}, NH(C=O)(CH₂)ₘNR⁴R^{4'}, CH₂O(CH₂)ₘNR⁴R^{4'}, CH₂OR⁴ ou COOR⁴,
R³ désigne H, A ou Hal,
R⁴ R^{4'} désignent chacun, indépendamment l'un de l'autre, H ou A,
R⁵ désigne CN, COOA ou COOH,
X, Y désignent chacun, indépendamment l'un de l'autre, O, NH, CH₂ ou sont absents,
A désigne alkyle non ramifié ou ramifié ayant 1-10 atomes de C, où 1-7 atomes de H peuvent être remplacés par F, ou cycloalkyle ayant 3-7 atomes de C,
Hal désigne F, Cl, Br ou I,
m désigne 1, 2 ou 3,
n désigne 0, 1 ou 2,
et des solvats, sels et stéréoisomères pharmaceutiquement utilisables de ceux-ci, y compris des mélanges de ceux-ci en toutes proportions.

2. Composés selon la revendication 1, dans lesquels
A désigne alkyle non ramifié ou ramifié ayant 1, 2, 3, 4, 5 ou 6 atomes de C, où 1-5 atomes de H peuvent être remplacés par F,
et des solvats, sels et stéréoisomères pharmaceutiquement utilisables de ceux-ci, y compris des mélanges de ceux-ci en toutes proportions.

3. Composés selon la revendication 1 ou 2, dans lesquels
R, R' désignent chacun, indépendamment l'un de l'autre, A ou Hal,
et des solvats, sels et stéréoisomères pharmaceutiquement utilisables de ceux-ci, y compris des mélanges de ceux-ci en toutes proportions.

4. Composés selon l'une ou plusieurs parmi les revendications 1-3, dans lesquels
R¹, R^{1'} désignent chacun, indépendamment l'un de l'autre, H ou F,
et des solvats, sels et stéréoisomères pharmaceutiquement utilisables de ceux-ci, y compris des mélanges de ceux-ci en toutes proportions.

5. Composés selon l'une ou plusieurs parmi les revendications 1-4, dans lesquels
R², R^{2'} désignent chacun, indépendamment l'un de l'autre, H, A ou OA,
et des solvats, sels et stéréoisomères pharmaceutiquement utilisables de ceux-ci, y compris des mélanges de ceux-ci en toutes proportions.

6. Composés selon l'une ou plusieurs parmi les revendications 1-5, dans lesquels
R⁴, R^{4'} désignent chacun, indépendamment l'un de l'autre, H ou CH₃,
et des solvats, sels et stéréoisomères pharmaceutiquement utilisables de ceux-ci, y compris des mélanges de ceux-ci en toutes proportions.

7. Composés selon l'une ou plusieurs parmi les revendications 1-6, dans lesquels
R³ désigne H,
et des solvats, sels et stéréoisomères pharmaceutiquement utilisables de ceux-ci, y compris des mélanges de ceux-ci en toutes proportions.

8. Composés selon l'une ou plusieurs parmi les revendications 1-6, dans lesquels
R⁵ désigne CN,
et des solvats, sels et stéréoisomères pharmaceutiquement utilisables de ceux-ci, y compris des mélanges de ceux-ci en toutes proportions.

9. Composés selon l'une ou plusieurs parmi les revendications 1-8, dans lesquels
X désigne O,
et des solvats, sels et stéréoisomères pharmaceutiquement utilisables de ceux-ci, y compris des mélanges de ceux-ci en toutes proportions.

10. Composés selon l'une ou plusieurs parmi les revendications 1-9, dans lesquels
Y est absent,
et des solvats, sels et stéréoisomères pharmaceutiquement utilisables de ceux-ci, y compris des mélanges de ceux-ci en toutes proportions.

11. Composés selon l'une ou plusieurs parmi les revendications 1-10, dans lesquels
R, R' désignent chacun, indépendamment l'un de l'autre, A ou Hal,
R¹, R^{1'} désignent chacun, indépendamment l'un de l'autre, H ou F,
R² R^{2'} désignent chacun, indépendamment l'un de l'autre, H, A ou OA,
R³ désigne H,
R⁴ R^{4'} désignent chacun, indépendamment l'un de l'autre, H ou CH₃,
R⁵ désigne CN,
X désigne O,
Y est absent,
A désigne alkyle non ramifié ou ramifié ayant 1, 2, 3, 4, 5 ou 6 atomes de C, où 1-5 atomes de H peuvent être remplacés par F,
Hal désigne F, Cl, Br ou I,
n désigne 1,
et des solvats, sels et stéréoisomères pharmaceutiquement utilisables de ceux-ci, y compris des mélanges de ceux-ci en toutes proportions.

12. Composés selon la revendication 1, choisis dans le groupe constitué par
| n° | Nom |
|---|---|
| "A1" | 2-{2-[1-Cyano-3-(2,6-diméthylphénylamino)indolizin-2-yl]-3-méthoxyphénoxy}acétamide |
| "A2" | 2-{2-[1-Cyano-3-(2,6-diméthylphénylamino)indolizin-2-yl]phénoxy}acétamide |
| "A3" | 2-{2-[1-Cyano-3-(2,6-diméthylphénylamino)indolizin-2-yl]-5-méthoxy-3-méthylphénoxy}acétamide |
| "A4" | 2-{2-[1-Cyano-3-(2,6-diméthylphénylamino)-6-fluoro-indolizin-2-yl]phénoxy}acétamide |
| "A5" | 2-{2-[1-Cyano-3-(2,6-dichlorophénylamino)indolizin-2-yl]phénoxy}acétamide |
| "A6" | 2-{2-[1-Cyano-3-(2-chloro-6-méthylphényl-amino)indolizin-2-yl]phénoxy}acétamide |
| "A7" | 2-{2-[1-Cyano-3-(2-éthyl-6-méthylphénylamino)indolizin-2-yl]phénoxy}acétamide |
| "A8" | 2-{2-[1-Cyano-3-(2,6-diméthylphénylamino)-6,8-difluoroindolizin-2-yl]phénoxy}acétamide |
| "A9" | 2-{2-[1-Cyano-3-(2,6-diméthylphénylamino)-6-fluoro-indolizin-2-yl]-3-méthylphénoxy}acétamide |
| "A10" | 2-{2-[1-Cyano-3-(2,6-diméthylphénylamino)-6-fluoro-indolizin-2-yl]-3-méthoxyphénoxy}acétamide |
| "A11" | 2-{2-[1-Cyano-3-(2,6-diméthylphénylamino)indolizin-2-yl]-3,5-diméthoxyphénoxy}acétamide |
| "A12" | 2-{2-[1-Cyano-3-(2,6-diméthylphénylamino)indolizin-2-yl]-3-éthylphénoxy}acétamide |
| "A13" | 2-{2-[1-Cyano-3-(2,6-diméthylphénylamino)-6-fluoro-indolizin-2-yl]-5-méthoxyphénoxy}acétamide |
| "A14" | 2-{2-[1-Cyano-3-(2,6-diméthylphénylamino)-6,8-difluoroindolizin-2-yl]-3-méthoxyphénoxy}acétamide |
| "A15" | 2-{2-[1-Cyano-3-(2,6-diméthylphénylamino)-6,8-difluoroindolizin-2-yl]-3-éthylphénoxy}acétamide |
| "A16" | 2-{2-[1-Cyano-3-(2,6-diméthylphénylamino)-6-fluoro-indolizin-2-yl]-3-éthylphénoxy}acétamide |
| "A17" | 2-{2-[1-Cyano-3-(2,6-diméthylphénylamino)-6-fluoro-indolizin-2-yl]-3,5-diméthoxyphénoxy}acétamide |
et des solvats, sels et stéréoisomères pharmaceutiquement utilisables de ceux-ci, y compris des mélanges de ceux-ci en toutes proportions.

13. Procédé de préparation de composés de formule I selon les revendications 1-12 et de solvats, sels et stéréoisomères pharmaceutiquement utilisables de ceux-ci, **caractérisé en ce que**
a) un composé de formule II 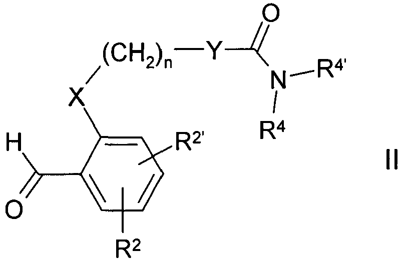 dans laquelle
X, Y, R², R^{2'}, R⁴, R^{4'} et n revêtent les significations indiquées selon la revendication 1,
est réagi avec un composé de formule III 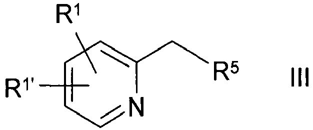 dans laquelle
R¹, R^{1'} et R⁵ revêtent les significations indiquées selon la revendication 1,
et avec un composé de formule IV 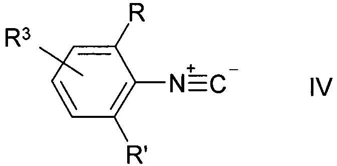 dans laquelle R, R' et R³ revêtent les significations indiquées selon la revendication 1,
et/ou
une base ou un acide de formule I est converti(e) en l'un de ses sels.

14. Médicaments comprenant au moins un composé de formule I selon l'une ou plusieurs parmi les revendications 1 à 12, et/ou des solvats, sels et stéréoisomères pharmaceutiquement utilisables de celui-ci, y compris des mélanges de ceux-ci en toutes proportions, et éventuellement des excipients et/ou des adjuvants.

15. Médicaments comprenant au moins un composé de formule I selon l'une ou plusieurs parmi les revendications 1 à 12, et/ou des solvats et stéréoisomères pharmaceutiquement utilisables de celui-ci, y compris des mélanges de ceux-ci en toutes proportions, et au moins un autre composé actif médicamenteux.

16. Utilisation de composés selon l'une ou plusieurs parmi les revendications 1 à 12, et/ou de sels et solvats physiologiquement acceptables de ceux-ci, pour la préparation d'un médicament destiné au traitement du diabète de type 1 et de type 2.

17. Utilisation de composés selon l'une ou plusieurs parmi les revendications 1 à 12, et/ou de sels et solvats physiologiquement acceptables de ceux-ci, pour la préparation d'un médicament destiné à abaisser la glycémie.

18. Utilisation de composés selon l'une ou plusieurs parmi les revendications 1 à 12, et/ou de sels et solvats physiologiquement acceptables de ceux-ci, et d'un autre composé actif médicamenteux, pour la préparation d'un médicament destiné au traitement du diabète de type 1 et de type 2.

19. Ensemble (kit) constitué de conditionnements séparés
(a) d'une quantité efficace d'un composé de formule I selon l'une ou plusieurs parmi les revendications 1 à 12, et/ou de solvats, sels et stéréoisomères pharmaceutiquement utilisables de celui-ci, y compris des mélanges de ceux-ci en toutes proportions,
et
(b) d'une quantité efficace d'un autre composé actif médicamenteux.

20. Utilisation de composés selon l'une ou plusieurs parmi les revendications 1 à 12, et/ou de sels et solvats physiologiquement acceptables de ceux-ci et d'un autre composé actif médicamenteux, pour la préparation d'un médicament destiné à abaisser la glycémie.
